(19) Europäisches Patentamt
European Patent Office
Office européen des brevets

(11) Publication number: 0 342 924 B1

(12) EUROPEAN PATENT SPECIFICATION

(45) Date of publication of patent specification: 11.10.95

(51) Int. Cl.[6]: A01N 47/36, A01N 47/34, A01N 47/28, A61K 7/50, A61K 7/48, A61K 7/42, A61K 7/075, A61K 7/06, A61K 7/02, A61L 9/01, C11D 9/50, //(A01N47/36,37:40, 37:38,37:10),(A01N47/34,37:40, 37:38,37:10),(A01N47/28,37:40, 37:38,31:10)

(21) Application number: 89304928.8

(22) Date of filing: 16.05.89

# (54) Stabilizer composition and stabilized aqueous systems.

(30) Priority: 16.05.88 US 194489
21.06.88 US 209516
02.05.89 US 346474

(43) Date of publication of application:
23.11.89 Bulletin 89/47

(45) Publication of the grant of the patent:
11.10.95 Bulletin 95/41

(84) Designated Contracting States:
AT BE CH DE ES FR GB GR IT LI LU NL SE

(56) References cited:
EP-A- 0 194 466
WO-A-81/00566
FR-A- 2 531 336
US-A- 3 248 285

(73) Proprietor: DALECO/PROTECH PARTNERS, L.P.
3333 W. Pacific Coast Highway,
The Harbour Club, 4th Floor
Newport Beach, California 92633 (US)

(72) Inventor: Rho, Jinnque
36 Whitney Lane
Orange, CT 06477 (US)
Inventor: Ghirla, Peter J.
324 Rockrimmon Road
Stamford, CT 06903 (US)

(74) Representative: Holdcroft, James Gerald, Dr. et al
Graham Watt & Co.,
Riverhead
Sevenoaks, Kent TN13 2BN (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

## Description

### Field of the Invention

This invention relates to improved stabilizer compositions for stabilizing aqueous systems, especially aqueous based enzyme compositions against bacterial contamination and to aqueous based systems stabilized against bacterial contamination.

### Background of the Invention

Water based compositions for a wide variety of purposes are well known, such as for example, aqueous based compositions useful as shampoos, cleansing creams, cosmetics, eye preparations, body lotions, pharmaceuticals, physiological saline solutions, odor and stain eradicators, disinfectants, suppositories and the like. The production of such compositions is such that it is intended that bacterial contamination be eliminated by killing of the bacteria during processing and production of the product. However, because of the adaptability of bacteria, particularly Pseudomonas bacteria, and because its nutritional demands are modest, it can survive and multiply quite easily. This is especially true with water based enzyme compositions where problems with the presence and growth of gram positive and gram negative bacteria severely limit the stability period of shelf life of the compositions to a period of about three to four months.

It has been discovered that the aforedescribed microbial contamination problem is especially prevalent in water based enzyme containing products due to contamination and growth of Pseudomonas aeruginosa. As a result of the aforesaid microbial contamination problem in water based enzyme containing products the marketing and use of such products has been inhibited.

Moreover, the presence and growth of bacterial contamination in the various types of water based products described hereinbefore can present a health hazard to those who utilize such products and incur the risk of bacterial infection. Severe health problems can result to those who utilize such contaminated products and are especially susceptible to contracting bacterial infections, such as chronically ill or severely debilitated persons and those on antibiotics or immunosuppressive therapy.

While stabilizers against such bacterial contaminations have heretofore been proposed, such stabilizers have not been particularly satisfactory or of sufficient efficacy to produce products of relatively long-term stability. Since the aforementioned compositions are often utilized in a manner that they require contact with skin or mucous membranes of humans or animals, it is necessary to utilize stabilizers or preservatives that are nontoxic, free or substantially free of harmful side effects and are environmentally safe to humans and animals. However, such stabilizers which have the aforesaid desirable characteristics have not been able to provide sufficient effective stabilization to water based compositions to enable the products to be stable over extended periods of time and during constant use.

It is therefore highly desirable that such water based compositions be stabilized so that the risk of bacterial infection from such products be eliminated or substantially eliminated and that such products have a increased shelf life or stability. It is also desirable that water based enzyme compositions be stabilized against bacterial contamination to produce water based enzyme compositions having a shelf life or stability of about two years or more and that such stabilized compositions be environmentally acceptable and safe to humans and animals who are exposed to or contact such compositions.

### Summary of the Invention

It has been discovered that aqueous based compositions of greatly improved stability with respect to bacterial contamination can be obtained by the addition thereto of a synergistic stabilizing amount of

a. sodium benzoate, and

b. a stabilizing effective amount of a urea compound selected from an optionally alkylolated diazolidinyl urea and an optionally substituted imidazolidinyl urea, or

c. the interaction product of a. and b.

The invention embraces a concentrated aqueous composition suitable for use in aqueous solutions subject to bacterial contamination composition comprising a mixture of or the interaction product of

a. sodium benzoate, and

b. a stabilizing effective amount of a urea compound selected from an optionally alkylolated diazolidinyl urea and an optionally alkylolated imidazolidinyl urea.

In the most preferred embodiment, the invention relates to aqueous based compositions of greatly improved stability with respect to bacterial contamination containing a synergistic stabilizing amount of

a. sodium benzoate and

b. a methylolated diazolidinyl urea of the formula

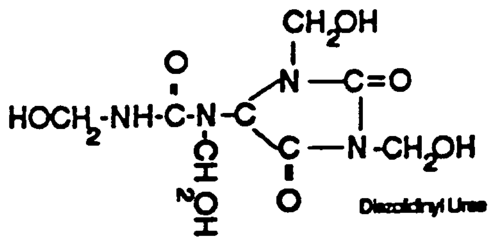

Diazolidinyl Urea

and/or a methylolated imidazolidinyl urea of the formula

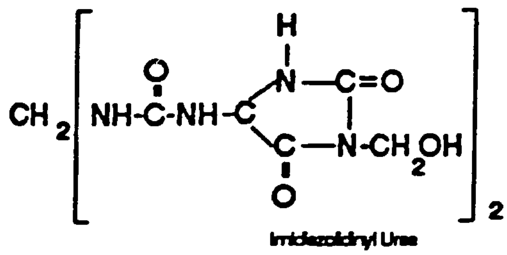

Imidazolidinyl Urea

## Detailed Description of the Invention

The invention is concerned with the stabilization of systems useful against such bacterial contaminations. More particularly, the invention relates to the stabilization of aqueous compositions for a wide variety of purposes, such as for example, aqueous based compositions useful as shampoos, cleansing creams, cosmetics, eye preparations, body lotions, pharmaceuticals, physiological saline solutions, odor and stain eradicators, disinfectants, suppositories and the like. This invention overcomes difficulties inherent in prior art systems which purport to provide stability from bacterial contamination. Coupled with the problem of stabilization is the fact that useful compositions are often utilized in a manner that they require contact with skin or mucous membranes of humans or animals, and this invention addresses that by providing a stabilizer system that has been established as nontoxic, free or substantially free of harmful side effects and environmentally safe to humans and animals.

The stabilizer system of the invention utilizes:

i. sodium benzoate, and

ii. a potentiating amount of an optionally alkylolated urea compound selected from an optionally alkylolated diazolidinyl urea and an optionally alkylolated imidazolidinyl urea, or

iii. the interaction product of i. and ii.

The term "interaction product" as used herein means the association of i. and ii. by molecular attraction ranging from strong to weak bonding relationships, including without limitation, covalent bonding, ionic bonding, Van der Waal forces, hydrogen bonding and/or associative bonding.

The optionally alkylolated urea compounds are water soluble compounds and include alkylolated ureas of the following structures:

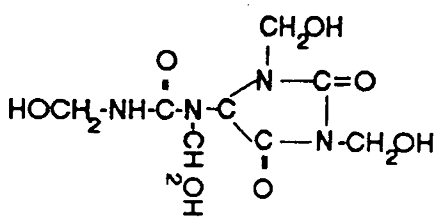

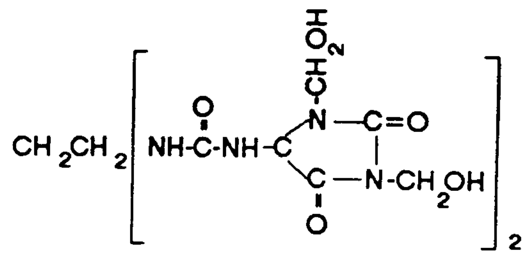

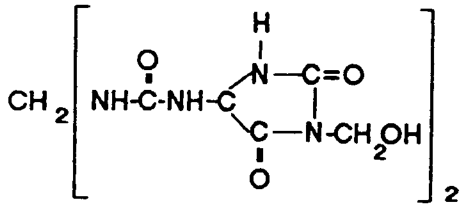

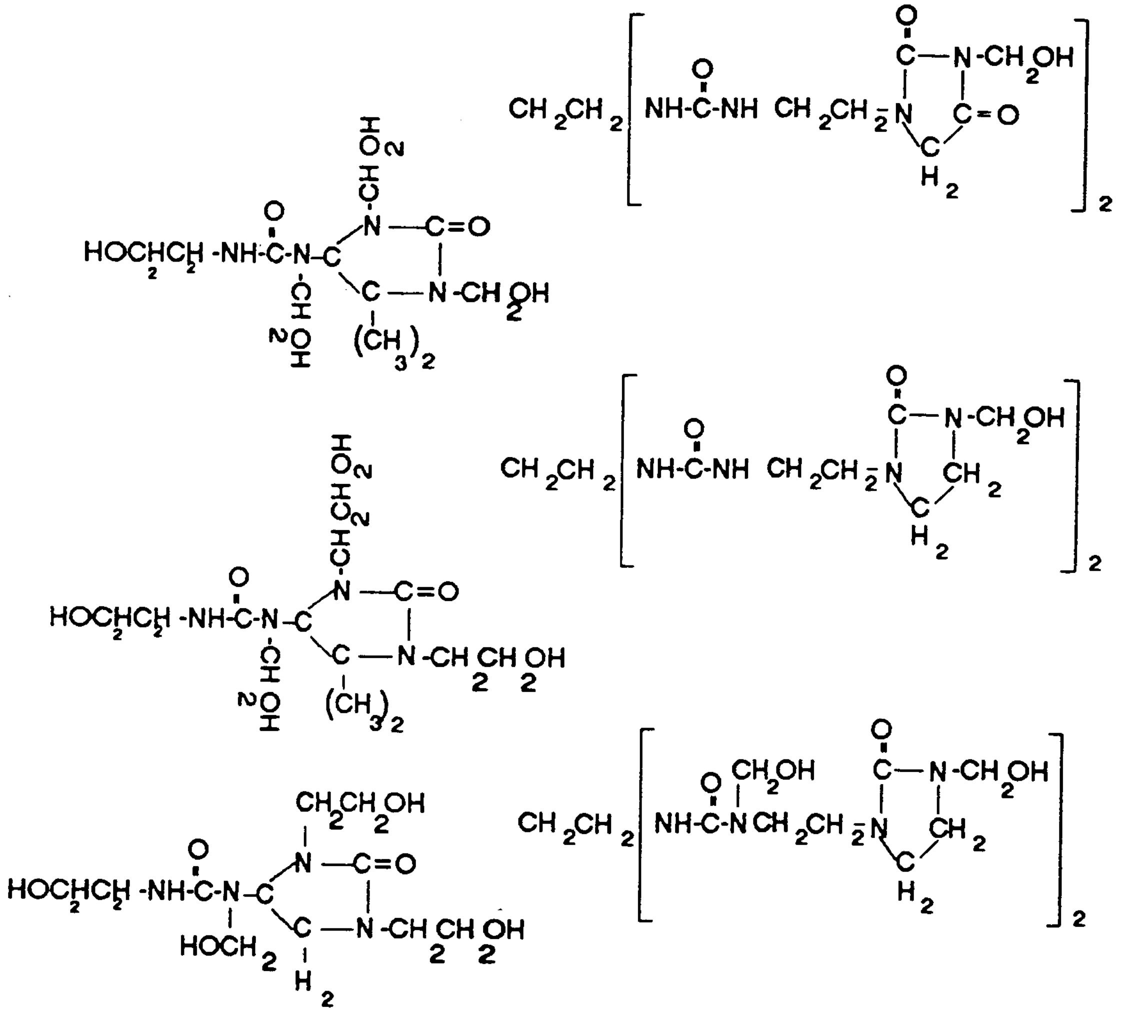

The most preferred compositions of the invention encompass aqueous based compositions of greatly improved stability with respect to bacterial contamination containing a synergistic stabilizing amount of

a. sodium benzoate, and

b. a methylolated diazolidinyl urea (hereinafter called "diazolidinyl urea") of the formula

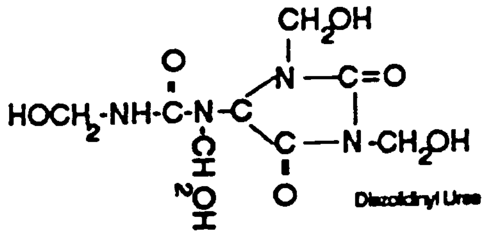

Diazolidinyl Urea

and/or a methylolated imidazolidinyl urea (hereinafter called "imidazolidinyl urea") of the formula

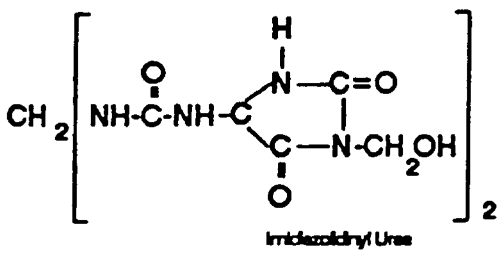

Imidazolidinyl Urea

These most preferred compositions provide water based compositions that are so well stabilized that the risks of bacterial infection from their use are eliminated, essentially eliminated or substantially eliminated over extended periods of time, resulting in products that have a significant, as well as unpredictable, increase in shelf life or stability. These most preferred compositions allow the production of water based enzyme compositions that are stabilized against bacterial contamination and have a shelf life or stability of about two years or more. Moreover, such stabilized compositions are environmentally acceptable and safe to humans and animals who are exposed to or contact them.

Mixed ureas are included within the scope of the invention.

The stabilization of such aqueous based compositions is much greater than is possible with either sodium benzoate or the urea compound alone, that is, the use of both components results in a potentiation of the stabilization obtained. It has also been discovered that the use of such synergistic stabilizing amounts is especially effective in stabilizing water based enzyme compositions where, heretofore, the problem of instability due to bacterial contamination has been especially pronounced.

The stabilisation of such aqueous based compositions, based on current evidence, is the most pronounced, within the scope of the invention, with the use of sodium benzoate and diazolidinyl urea and/or imidazolidinyl urea. In this case as well, but much greater than foreseeable, the use of sodium benzoate in combination with diazolidinyl urea and/or imidazolidinyl urea provides stabilization considerably superior to that obtainable from the use alone of sodium benzoate or diazolidinyl urea or imidazolidinyl urea. It has also been discovered that the use of such synergistic stabilizing amounts of sodium benzoate and diazolidinyl urea or imidazolidinyl urea is especially effective in stabilizing water based enzyme compositions where, heretofore, the problem of instability due to bacterial contamination has been especially pronounced.

The sodium benzoate and the urea compound diazolidinyl urea or imidazolidinyl urea stabilizer, can be added individually to the water based formulations in the appropriate amounts in order to obtain the desired stabilization. However, it is preferred that stabilizing compositions comprising mixtures of said two stabilizers be formed and that an appropriate effective stabilizing amount of a stabilizing composition be added to the water based systems.

The amounts of sodium benzoate and the urea compounds are not narrowly critical. Typically, they may be used in a weight ratio of from 0.1 to 10 of either sodium benzoate or the urea compound to the other. For example, such stabilizing compositions will generally comprise sodium benzoate and diazolidinyl urea or imidazolidinyl urea in a weight ratio of from 0.5:1 to 1:0.5, most preferably in a weight ratio of about 1:1. The optimum stabilizing composition comprises a 1:1 mixture of sodium benzoate and diazolidinyl urea or imidazolidinyl urea.

The amount of such stabilizing composition added to water based compositions to stabilize such water based compositions against bacterial contamination will be any bacterial effective stabilizing amount, generally from 0.05 to 2%, preferably 0.1 to 1% and most preferably 0.2 to 0.5% weight percent of stabilizing composition based on the total weight of the water based composition. It will be appreciated that the amount of stabilizing composition incorporated in the water based compositions will vary with the type and components of the water based compositions.

The amount of each stabilizing component employed in the water based compositions to stabilize said compositions against bacterial contamination will generally be in a amount of from 0.025% to 1%, preferably 0.05% to 0.5% and most preferably from 0.1% to 0.25%, and most preferably about 0.1% by weight of each of sodium benzoate and the urea compounds, such as diazolidinyl urea or imidazolidinyl urea.

The unique synergistic antibacterial effective stabilizer combination of sodium benzoate, and the urea compound, e.g., diazolidinyl urea or imidazolidinyl urea, may be employed to stabilize a wide variety of water based compositions where stabilization against bacterial contamination is a problem. As examples of such water based compositions in need of stabilization and which may be stabilized by the combination of stabilizers of this inventions, there may be mentioned, for example, the following type water based compositions: pharmaceuticals, cosmetics, odor and stain eradicators, ointments, bathing lotions, skin astringents, sunscreens, shampoos, hair treatment products such as hair conditions and permanent compositions, mousse removers, eye preparations, disinfectants, makeup removers, cleansing creams and lotions. The combination of stabilizers has been found to be particularly effective stabilizers for water based enzyme containing odor and stain eradicating compositions where stability of such compositions against bacterial contamination has been an especially prevalent need in order to increase the stability term or shelf life of such products.

The stabilizing compositions, the stabilized water based compositions and the process of stabilizing water based compositions against bacterial contamination is illustrated but not limited by the following examples.

**Example 1**

A stabilizing composition of this invention comprises a 1:1 weight ratio mixture of sodium benzoate and diazolidinyl urea or imidazolidinyl urea. Other stabilizing compositions of this invention comprise the mixtures of sodium benzoate with diazolidinyl urea or imidazolidinyl urea in weight ratios of 0.5:1, 1:0.5, 0.75:1 and 1:0.75.

**Example 2**

A commercially available enzyme containing water based product, VETCAIR™, from Protech Inc., Stamford, Connecticut, was stabilized according to this invention and found to be stabilized against bacterial contamination in an unexpectedly superior synergistic manner from the combined use of sodium benzoate and imidazolidinyl urea compared to the use of either component alone. The VETCAIR™ product is an enzyme based product for neutralization or eradication of odors and prevention of stains due to animals such as dogs, cats, rabbits, ferrets, monkeys, and horses. The VETCAIR™ product is an enzyme containing water based product containing lyase, isomerase, ligase, oxidoreductase, transferase and hydrolase enzymes. Control A consisted of VETCAIR™ with no stabilizer added. Control B consisted of VETCAIR™ with 0.1% by weight sodium benzoate added and Control C consisted of VETCAIR™ with 0.1% imidazolidinyl urea added. For comparison purposes, two further control compositions were prepared, namely Control D consisting of VETCAIR™ with 0.1% propyl parahen added, Control E consisting of VETCAIR™ with 0.1% propyl paraben and 0.1% sodium benzoate.

A composition according to this invention was prepared and consisted of VETCAIR™ with 0.1% sodium benzoate and 0.1% imidazolidinyl urea added, designated Composition F.

All the compositions were tested for preservative or stabilization effectiveness according to the following two test procedures using two Pseudomonas aeruginosa product isolates identified as Q211-B and Q211-Y. Both cultures of the Pseudomonas product isolates were subcultured in Trypticase Soy Broth (TSB). The cultures were incubated for 24 hours at 30 ° C. One-tenth ml of 24 hour culture of each organism was used to challenge of the compositions.

The two test methods employed were as follows:

1. Challenge Test: The challenge test used was a modification of the Standard Cosmetic, Toiletries, and Fragrance Association Procedure. The products were challenged with the product isolates. Challenges

were made with pure cultures. The challenge levels were $10^5$-$10^6$/ml. All product compositions were inoculated with 0.1 ml of inoculum and reinoculated with the same amount 7 days later. Aliquots of inoculated formulations were taken aseptically to allow determination of the number of microorganisms by a count of colonies after standing 4, 7, 14 and 28 days at 30 ° C.

2. Repeated inoculation-incubation test: Duplicate samples were prepared as described in the challenge test. Samples were restreaked on Trypticase Soy Agar (TSA) plus 1.5% Tween 80 after 24 hours incubation. The compositions samples and streak plates were then incubated at 30 ° C. Plates were read after 48 hours incubation. Samples were reinoculated after another 24 hours incubation at 30 ° C (48 hours total incubation time between inoculations), and again streaked 24 hours later.

This procedure was repeated for a total of ten inoculation-incubation cycles. A stabilizer or preservative was considered to give adequate in-use protection only if it maintained sterility in the test sample throughout all ten inoculation-incubation cycles.

To quantitatively determine the stabilization effectiveness serial tenfold dilutions of challenged compositions from $10^{-1}$-$10^{-6}$ were made in phosphate buffer; 0.1 ml aliquots were removed from each dilution and placed on the surface of the TSA + Tween 80 plate. A sterile glass rod was used to streak the sample uniformly over the plate. Counts were made and recorded as colony forming unit (CFU)/ml.

The results of the bacterial challenge test (Test 1) are set forth in Table 1.

**Table 1**

| COMPOSITION | P.aeruginosa Q211-B Days after | | | | CFU/ml P.aeruginosa Q211-Y Days after | | | |
|---|---|---|---|---|---|---|---|---|
| | 4 | 7 | 14 | 28 | 4 | 7 | 14 | 28 |
| Control A | <10 | $2x10^5$ | $4.2x10^6$ | $1.3x10^8$ | - | - | - | - |
| Control B | <10 | $2.5x10^5$ | $3.5x10^6$ | $7.0x10^7$ | - | - | - | - |
| Control C | <10 | <10 | <10 | <10 | <10 | <10 | $3x10^5$ | $2x10^5$ |
| Control D | <10 | <10 | $2x10^6$ | $1.8x10^6$ | <10 | <10 | $5x10^4$ | $2.5x10^6$ |
| Control E | <10 | $9.8x10^4$ | $3.9x10^6$ | $2.6x10^7$ | <10 | <10 | $2x10^5$ | $1.6x10^7$ |
| Control F | <10 | <10 | <10 | <10 | <10 | <10 | <10 | <10 |

All samples were reinoculated with 0.1 ml of inoculum at day 7.
Numbers represent colony forming unit/ml (CFU/ml).

The test results of the challenge test in Table 1 indicate that only Composition F containing both sodium benzoate and imidazolidinyl urea exhibited effective bacterial contamination control by rapidly reducing and essentially eliminating the contaminating bacterial inoculums in the products.

Test 2, the repeated inoculation-incubation test, is a test indicative of the product composition maintaining control of microorganisms during repeated or constant use. The results of this test are set forth in Table 2.

Table 2

| Day | A | B | C | D | E | F |
|---|---|---|---|---|---|---|
| 1 | - | + | - | - | - | - |
| 3 | + | + | - | - | - | - |
| 5 | + | + | - | - | - | - |
| 7 | + | + + | - | + | + | - |
| 9 | + + | + + | + | + | + | - |
| 11 | + + + | + + + | + | + | + + + | - |
| 15 | + + + | + + + | + | + + | + + + | - |
| 28 | + + + | + + + | + + | + + + | + + + | - |
| - No growth + Slight growth<br>+ + Moderate growth + + + Heavy growth | | | | | | |

The results of this repeated insult test indicate that of the tested compositions only Composition F provides effective protection to control growth of P. aeruginosa microorganisms during constant use.

Substitution of diazolidinyl urea in place of imidazolidinyl was in Compositions C and F in each of the aforesaid challenge tests and repeated inoculation - incubation tests produce similar stabilization results.

In addition to stabilizing and preserving water based compositions against growth of P. aeruginosa as set forth hereinbefore, the stabilizer compositions of this invention have also been shown to be effective stabilizing and preserving water based compositions against the following organisms: Staphylococcus aureus, Escherichia coli, Aspergillus niger, Candida albicans and Bacillus cereus.

The combination of sodium benzoate and diazolidinyl urea not only synergistically protects water based compositions against microbial contamination, but is considered environmentally acceptable and safe to humans and animals.

Though this invention has been described in the examples with respect to specific materials, it is not intended that the invention be limited thereto. The invention contemplates the use of a variety of materials encompassed by the description herein set forth.

## Claims

**Claims for the following Contracting States : AT, BE, CH, DE, FR, GB, IT, LI, LU, NL, SE**

1. A stabilizer composition for protecting aqueous based compositions against microbial growth comprising sodium benzoate and a stabilizing effective amount of a urea compound selected from an optionally alkylolated diazolidinyl urea and an optionally alkylolated imidazolidinyl urea.

2. A stabilizer composition according to claim 1 comprising sodium benzoate and a urea compound in a weight ratio of from 0.5:1 to 1:05.

3. A stabilizer composition according to claim 1 or claim 2 comprising sodium benzoate and a urea compound in a weight ratio of 1:1.

4. A composition according to any one of claims 1 to 3, wherein the urea compound is selected from (a) a methylolated diazolidinyl urea of the formula (III)

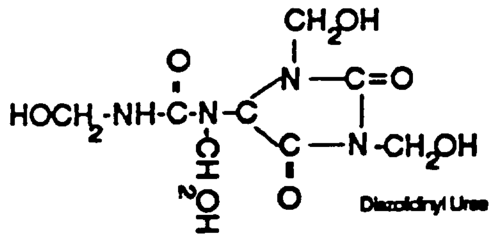

Diazolidinyl Urea (III)

and (b) a methylolated imidazolidinyl urea of the formula (IV)

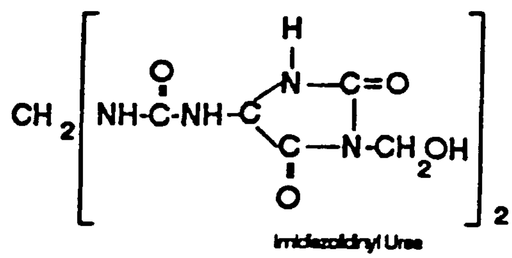

Imidazolidinyl Urea (IV)

5. An aqueous based composition stabilized against microbial growth comprising an aqueous based composition and an effective amount of a stabilizer composition according to any one of claims 1 to 4.

6. A composition according to claim 5, wherein the amount of sodium benzoate present is from 0.05% to 0.5% by weight and the amount of urea compound present is from 0.05% to 0.5%.

7. A composition according to claim 5 or claim 6, wherein the amount of sodium benzoate present is 0.1% by weight and the amount of urea compound present is 0.1% by weight.

8. A composition according to any one of claims 5 to 7, wherein the aqueous based composition is one of the following: pharmaceuticals, cosmetics, odor and stain eradicators, ointments, bathing lotions, skin astringents, sunscreens, shampoos, hair treatment products, mousse removers, eye preparations, disinfectants, make-up removers and cleansing creams or lotions.

9. A composition according to any one of claims 5 to 8, wherein the aqueous based composition is an enzyme containing aqueous based composition.

10. A composition according to claim 9, wherein the enzyme containing aqueous based composition is an odor or stain eradicator containing a mixture of lyase, isomerase, ligase, oxidoreductase, transferase and hydrolase enzymes.

**Claims for the following Contracting States : ES, GR**

1. A method of making a stabilizer composition for protecting aqueous based compositions against microbial growth comprising forming a mixture of sodium benzoate and a stabilizing effective amount of a urea compound selected from an optionally alkylolated diazolidinyl urea and an optionally alkylolated imidazolidinyl urea.

2. A method according to claim 1, wherein the composition comprises sodium benzoate and a urea compound in a weight ratio of from 0.5:1 to 1:05.

3. A method according to claim 1 or claim 2, wherein the composition comprises sodium benzoate and a urea compound in a weight ratio of 1:1.

4. A method according to any one of claims 1 to 3, wherein the urea compound is selected from (a) a methylolated diazolidinyl urea of the formula (III)

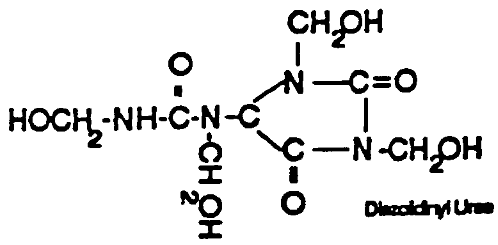

Diazolidinyl Urea (III)

and (b) a methylolated imidazolidinyl urea of the formula (IV)

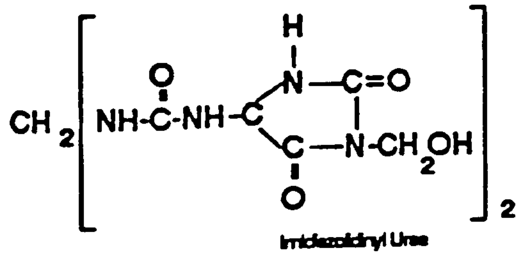

(IV)

Imidazolidinyl Urea

5. A method of making an aqueous based composition stabilized against microbial growth comprising forming a mixture of an aqueous based composition and an effective amount of a stabilized composition made by a method according to any one of claims 1 to 4.

6. A method according to claim 4, wherein the amount of sodium benzoate present is from 0.05%, to 0.5% by weight and the amount of urea compound present is from 0.05% to 0.5%.

7. A method according to claim 5 or claim 6, wherein the amount of sodium benzoate present is 0.1% by weight and the amount of urea compound present is 0.1% by weight.

8. A method according to any one of claims 5 to 7, wherein the aqueous based composition is one of the following: pharmaceuticals, cosmetics, odor and stain eradicators, ointments, bathing lotions, skin astringents, sunscreens, shampoos, hair treatment products, mousse removers, eye preparations, disinfectants, make-up removers and cleansing creams or lotions.

9. A method according to any one of claims 5 to 8, wherein the aqueous based composition is an enzyme containing aqueous based composition.

10. A method according to claim 9, wherein the enzyme containing aqueous based composition is an odor or stain eradicator containing a mixture of lyase, isomerase, ligase, oxidoreductase, transferase and hydrolase enzymes.

**Patentansprüche**

**Patentansprüche für folgende Vertragsstaaten : AT, BE, CH, DE, FR, GB, IT, LI, LU, NL, SE**

1. Eine Stabilisator-Zusammensetzung zum Schutz von wasserhaltigen Zusammensetzungen gegen mikrobielles Wachstum enthaltend oder bestehend aus Natriumbenzoat und einem zur Stabilisierung wirksamen Gehalt an einer Harnstoffverbindung ausgewählt aus einem ggf. alkylolsubstituierten Diazolidinyl-Harnstoff und einem ggf. alkylol-substituierten Imidazolidinyl-Harnstoff.

2. Eine Stabilisator-Zusammensetzung gemäß Anspruch 1 enthaltend oder bestehend aus Natriumbenzoat und einer Harnstoffverbindung in einem Gewichtsverhältnis von 0,5:1 bis 1:0,5 .

**3.** Eine Stabilisator-Zusammensetzung gemäß Anspruch 1 oder Anspruch 2 enthaltend oder bestehend aus Natriumbenzoat und einer Harnstoffverbindung in einem Gewichtsverhältnis von 1:1 .

**4.** Eine Zusammensetzung gemäß einem der Ansprüche 1 bis 3, worin die Harnstoffverbindung ausgewählt ist aus
(a) einem methylol-substituierten Diazolidinyl-Harnstoff der Formel (III)

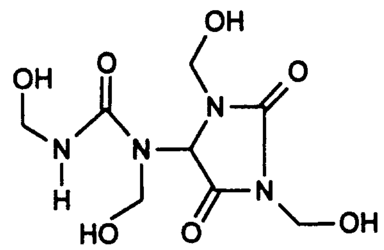

Diazolidinyl-Harnstoff (III)

und
(b) einem methylol-substituierten Imidazolidinyl-Harnstoff der Formel (IV)

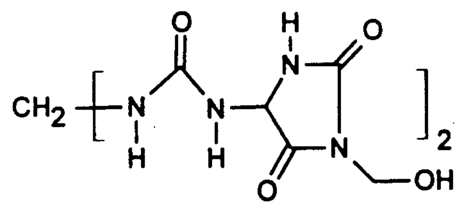

Imidazolidinyl-Harnstoff (IV)

**5.** Eine wasserhaltige Zusammensetzung, die gegen mikrobielles Wachstum stabilisiert ist, enthaltend oder bestehend aus einer wasserhaltigen Zusammensetzung und einem wirksamen Gehalt einer Stabilisator-Zusammensetzung gemäß einem der Ansprüche 1 bis 4.

**6.** Eine Zusammensetzung gemäß Anspruch 5, worin der Gehalt an Natriumbenzoat 0,05 bis 0,5 Gew.% und der Gehalt an Harnstoffverbindung 0,05 bis 0,5 Gew.% beträgt.

**7.** Eine Zusammensetzung gemäß Anspruch 5 oder Anspruch 6, worin der Gehalt an Natriumbenzoat 0,1 Gew.% und der Gehalt an Harnstoffverbindung 0,1 Gew.% beträgt.

**8.** Eine Zusammensetzung gemäß einem der Ansprüche 5 bis 7, worin die wasserhaltige Zusammensetzung eine der folgenden ist: Pharmazeutika, Kosmetika, Geruchs- und Fleckenentfernungsmittel, Salben, Badelotionen. Hautadstringentien, Sonnenschutzmittel, Schampoos. Haarbehandlungsprodukte, Mousse-Entferner, Augenpräparate, Desinfektionsmittel, Make-up-Entferner und Reinigungscremes oder-lotionen.

**9.** Eine Zusammensetzung gemäß einem der Ansprüche 5 bis 8, worin die wasserhaltige Zusammensetzung eine enzymenthaltende wasserhaltige Zusammmensetzung ist.

**10.** Eine Zusammmensetzung gemäß Anspruch 9, worin die enzym-enthaltende wasserhaltige Zusammensetzung ein Geruchs- oder Fleckenentfernungssmittel ist, das eine Mischung von Lyase-, Isomerase-, Ligase-,Oxidoreduktase-, Transferase- und Hydrolase-Enzymen enthält.

**Patentansprüche für folgende Vertragsstaaten : ES, GR**

1. Ein Verfahren zur Herstellung einer Stabilisator-Zusammensetzung zum Schutz von wasserhaltigen Zusammensetzungen gegen mikrobielles Wachstum, umfassend die Bildung einer Mischung aus Natriumbenzoat und einem zur Stabilisierung wirksamen Gehalt an einer Harnstoffverbindung ausgewählt aus einem ggf. alkylolsubstituierten Diazolidinyl-Harnstoff und einem ggf. alkylol-substituierten Imidazolidinyl-Harnstoff.

2. Ein Verfahren gemäß Anspruch 1, wobei die Zusammensetzung Natriumbenzoat und eine Harnstoffverbindung in einem Gewichtsverhältnis von
0,5:1 bis 1:0,5 enthalt oder daraus besteht.

3. Ein Verfahren gemäß Anspruch 1 oder Anspruch 2, wobei die Zusammensetzung Natriumbenzoat und eine Harnstoffverbindung in einem Gewichtsverhältnis von 1:1 enthält oder daraus besteht.

4. Ein Verfahren gemäß einem der Ansprüche 1 bis 3, wobei die Harnstoffverbindung ausgewählt ist aus
(a) einem methylol-substituierten Diazolidinyl-Harnstoff der Formel (III)

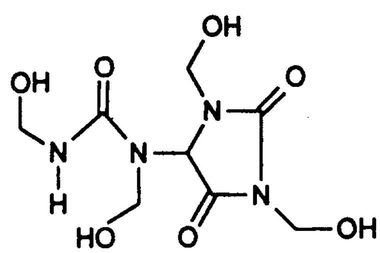

Diazolidinyl-Harnstoff (III)

und
(b) einem methylol-substituierten ImidazolidinylHarnstoff der Formel (IV)

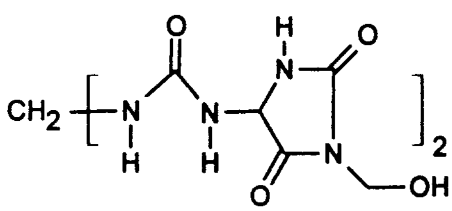

Imidazolidinyl-Harnstoff (IV).

5. Ein Verfahren zur Herstellung einer wasserhaltigen Zusammensetzung, die stabilisiert gegen mikrobiellen Wachstum ist, umfassend die Bildung einer Mischung aus einer wasserhaltigen Zusammensetzung und einem wirksamen Gehalt einer Stabilisator-Zusammensetzung gemäß einem der Verfahren der Ansprüche 1 bis 4.

6. Ein Verfahren gemäß Anspruch 5, wobei der Gehalt an anwesendem Natriumbenzoat 0,05 bis 0,5 Gew.% beträgt und der Gehalt an Harnstoffverbindung 0,05 bis 0,5 Gew.% beträgt.

7. Ein Verfahren gemäß Anspruch 5 oder Anspruch 6, wobei der Gehalt an anwesendem Natriumbenzoat 0,1 Gew.% beträgt und der Gehalt an Harnstoffverbindung 0,1 Gew.% beträgt.

8. Ein Verfahren gemäß einem der Ansprüche 5 bis 7, wobei die wasserhaltige Zusammensetzung eine der folgenden ist: Pharmazeutika, Kosmetika, Geruchs- und Fleckenentfernungsmittel, Salben, Badelotionen, Hautadstringentien, Sonnenschutzmittel, Schampoos, Haarbehandlungsprodukte, Mousse-Entferner, Augenpräparate, Desinfektionsmittel, Make-up-Entferner und Reinigungscremes oder -lotionen.

9. Ein Verfahren gemäß einem der Ansprüche 5 bis 8, wobei die wasserhaltige Zusammensetzung eine enzym-enthaltende wasserhaltige Zusammmensetzung ist.

10. Ein Verfahren gemäß Anspruch 9, wobei die enzym-enthaltende wasserhaltige Zusammmensetzung ein Geruchs- oder Fleckenentfernungsmittel ist, das eine Mischung von Lyase-, Isomerase-, Ligase-, Oxidoreduktase-, Transferase- und Hydrolase-Enzymen enthält.

**Revendications**
**Revendications pour les Etats contractants suivants : AT, BE, CH, DE, FR, GB, IT, LI, LU, NL, SE**

1. Composition stabilisante pour protéger des compositions à base aqueuse contre la croissance microbienne, comprenant du benzoate de sodium et une quantité efficace, eu égard au pouvoir stabilisant, d'un composé dérivé d'urée choisi parmi une diazolidinyl urée éventuellement alkylolée et une imidazolidinyl urée éventuellement alkylolée.

2. Composition stabilisante selon la revendication 1 comprenant du benzoate de sodium et un composé dérivé d'urée dans un rapport pondéral de 0,5:1 à 1:0,5.

3. Composition stabilisante selon la revendication 1 ou 2 comprenant du benzoate de sodium et un composé dérivé d'urée dans un rapport pondéral de 1:1.

4. Composition selon l'un quelconque des revendications 1 à 3, dans laquelle le composé dérivé d'urée est choisi parmi (a) une diazolidynil urée méthylolée de formule (III)

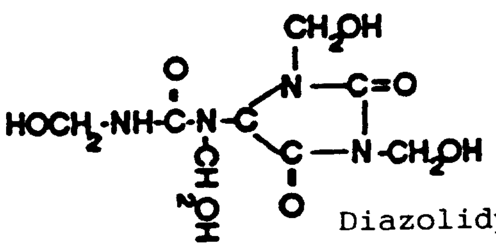

Diazolidynil urée (III)

et (b) une imidazolidynil urée méthylolée de formule (IV)

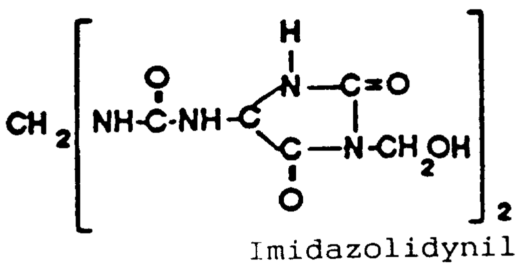

Imidazolidynil urée (IV)

5. Composition à base aqueuse stabilisée contre la croissance microbienne, comprenant une composition à base aqueuse et une quantité efficace d'une composition stabilisante selon l'une quelconque des revendications 1 à 4.

6. Composition selon la revendication 5, dans laquelle la teneur en benzoate de sodium présent est de 0,05 % à 0,5 % en poids et la teneur en composé dérivé d'urée présent est de 0,05 % à 0,5 %.

7. Composition selon la revendication 5 ou 6, dans laquelle la teneur en benzoate de sodium présent est de 0,1 % en poids et la teneur en composé dérivé d'urée présent est de 0,1 % en poids.

8. Composition selon l'une quelconque des revendications 5 à 7, dans laquelle la composition à base aqueuse est choisie parmi des compositions pharmaceutiques, cosmétiques, des compositions pour éliminer les odeurs et les taches, des pommades, des lotions pour le bain, des produits astringents pour la peau, des écrans solaires, des shampooings, des produits de traitement des cheveux, des produits pour éliminer la mousse, des préparations pour les yeux, des désinfectants, des démaquillants, et des crèmes ou lotions nettoyantes.

9. Composition selon l'une quelconque des revendications 5 à 8, dans laquelle la composition à base aqueuse est une composition à base aqueuse contenant une enzyme.

10. Composition selon la revendication 9, dans laquelle la composition à base aqueuse contenant une enzyme est une composition pour éliminer les odeurs ou les taches, contenant un mélange d'enzymes lyase, isomérase, ligase, oxydoréductase, transférase et hydrolase.

**Revendications pour les Etats contractants suivants : ES, GR**

1. Procédé de préparation d'un composition stabilisante pour protéger des compositions à base aqueuse contre la croissance microbienne, comprenant la préparation d'un mélange de benzoate de sodium et d'une quantité efficace, eu égard au pouvoir stabilisant, d'un composé dérivé d'urée choisi parmi une diazolidinyl urée éventuellement alkylolée et une imidazolidinyl urée éventuellement alkylolée.

2. Procédé selon la revendication 1, dans lequel la composition comprend du benzoate de sodium et un composé dérivé d'urée dans un rapport pondéral de 0,5:1 à 1:0,5.

3. Procédé selon la revendication 1 ou 2, dans lequel la composition comprend du benzoate de sodium et un composé dérivé d'urée dans un rapport pondéral de 1:1.

4. Procédé selon l'un quelconque des revendications 1 à 3, dans lequel le composé dérivé d'urée est choisi parmi (a) une diazolidynil urée méthylolée de formule (III)

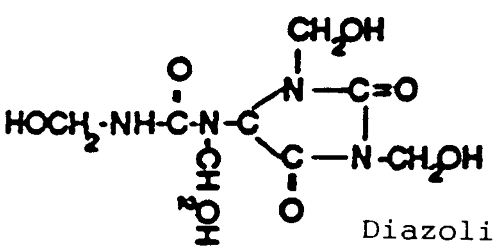

Diazolidynil urée (III)

et (b) une imidazolidynil urée méthylolée de formule (IV)

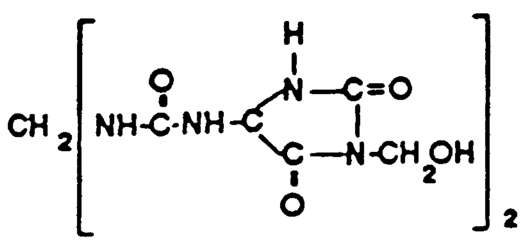

Imidazolidynil urée (IV)

5. Procédé de préparation d'une composition à base aqueuse stabilisée contre la croissance microbienne, comprenant la préparation d'un mélange d'une composition à base aqueuse et d'une quantité efficace d'une composition stabilisante préparée suivant un procédé selon l'une quelconque des revendications 1 á 4.

**6.** Procédé selon la revendication 4, dans lequel la teneur en benzoate de sodium présent est de 0,05 % à 0,5 % en poids et la teneur en composé dérivé d'urée présent est de 0,05 % à 0,5 %.

**7.** Procédé selon la revendication 5 ou 6, dans lequel la teneur en benzoate de sodium présent est de 0,1 % en poids et la teneur en composé dérivé d'urée présent est de 0,1 % en poids.

**8.** Procédé selon l'une quelconque des revendications 5 à 7, dans lequel la composition à baie aqueuse est choisie parmi des compositions pharmaceutiques, cosmétiques, des compositions pour éliminer les odeurs et les taches, des pommades, des lotions pour le bain, des produits astringents pour la peau, des écrans solaires, des shampooings, des produits de traitement des cheveux, des produits pour éliminer la mousse, des préparations pour les yeux, des désinfectants, des démaquillants, et des crèmes ou lotions nettoyantes.

**9.** Procédé selon l'une quelconque des revendications 5 à 8, dans lequel la composition à base aqueuse est une composition à base aqueuse contenant une enzyme.

**10.** Procédé selon la revendication 9, dans lequel la composition à base aqueuse contenant une enzyme est une composition pour éliminer les odeurs ou les taches, contenant un mélange d'enzymes lyase, isomérase, ligase, oxydoréductase, transférase et hydrolase.